# EUROPEAN PATENT APPLICATION

(11) **EP 1 870 064 A1**
(43) Date of publication of application: **26.12.2007**
(21) Application number: 07012153.8
(22) Date of filing: 21.06.2007
(51) Int. Cl.: A61F 13/08, A61H 33/14

(54) **Therapeutical equipment for treating aortic stenosis**

(30) Priority: 22.06.2006 HU 0600516
(71) Applicant: Darabos, János, Széchenyi u. 3. 4051 Hajdúsámson (HU)
(72) Inventor: Darabos, János, Széchenyi u. 3. 4051 Hajdúsámson (HU)
(74) Representative: Emri, Jozsefné

(57) **Abstract**

The object of the invention is a medical appliance for the treatment of mainly vasoconstriction, based on the vasodilator effect of carbon dioxide.

The solution according to the invention is a hose made of a flexible material not permeable to gas, loosely following the shape of the body, which can be fixed preferably near the top of the thigh or under the knee by means of a self-adhesive tape ensuring airtight sealing, where the space between the surface of the body and the hose is filled with carbon dioxide gas (2).

## Description

The object of the invention is a medical appliance for the treatment of mainly vasoconstriction, based on the vasodilator effect of carbon dioxide.

In Hungary one million people suffer from vascular diseases, and unfortunately there are three times more limb amputations than in Western-Europe.

Natural therapies play an increasing role in the treatment of vasoconstriction, in addition to or in stead of drug therapies. The most effective natural therapy for the treatment of vasoconstriction is a dry carbon dioxide bath, the so called mofette. Mofette is a geological term referring to: a relatively low-temperature volcanic flow with a carbon dioxide content of over 90% by volume. In Europe a natural gas bath is a rare phenomenon. Besides the 25-30 "therapeutic pits" in Transylvania (which have been known for centuries), there is a similar gas source in Bad Gastein, Austria, and also near Naples, the so called dog's cave, but there the CO₂ concentration is low.
In Hungary there is only one carbon dioxide flow, in Mátraderecske, and on the basis of announcement No. 14/1999 of the Ministry of Health (Official Journal of the Ministry of Health, issue No. 15), the gas of the carbon dioxide gas source, flowing naturally, as a natural therapeutic factor, is classified as a therapeutic gas for external application as a bath gas.
Here the treatment is given in a course under the supervision of physicians and qualified health care workers, a course consists of 15 treatments. The level of the colourless, odourless, poisonous CO₂ gas is indicated by an extinguishing candle and a balloon filled with oxygen. In the carbon dioxide bath, on the skin surfaces in contact with the gas the bather feels pleasant warmth, tingling, and the surface of the skin reddens slightly due to the widening of the blood vessels. As the gas diffusing through the skin widens the blood vessels through a reflex mechanism, it has an effect not only on the skin, but on deeper tissue layers, the connective tissue and the internal organs as well. The small arteries, arterioles open, which can be well demonstrated by a so called Doppler-test. As a result of the gas bath the contractions of the heart become stronger, breathing deepens, blood pressure decreases. The therapeutic gas is very suitable for the treatment of heart and peripheral artery diseases - thus for example lower limb vasoconstriction - high blood pressure, the blood vessel complications of diabetes, the conditions following vascular surgery, as well as in case of arthrosis and osteoporosis.
The beneficial effect of mofettes for patients with vasoconstriction has been demonstrated many times, but treatment at a mofette is quite expensive due to the additional costs (travel, accommodation, catering, treatment fee).

In the followings patent applications relating to the treatment of vasoconstriction using non-drug therapies are presented.

Patent No. 224572 entitled "Apparatus for the treatment of patients with vascular diseases with a combination of infra-, audible and ultrasound waves" discloses an invention the object of which is an apparatus which emits waves in such a power and frequency range that they can propagate into the deep layers of the body part to be treated, thus causing the target tissues, primarily the walls of muscular type arteries and arteries smaller than those to vibrate. As a result of the vascular massage, through increased diffusion in the walls of the blood vessels, biological processes regain their activity.

The field of application of the invention is the treatment of diseases caused by poor circulation, mainly vasoconstriction and decubitus.

Patent No. 220349 is based on the beneficial, vasodilator effect of carbonic acid. The latter patent describes an apparatus for preparing carbonic acid baths, making use of the therapeutic effect of carbonic acid hydrotherapy.
The disadvantages of this solution are that it can be used only under supervision, it is not reasonable to buy the apparatus for home use as carbonic acid bathing shall be applied in a course (what happens to the apparatus after the course), moreover, the treatment is made difficult by the fact that the bath tub/pool has to be covered during bathing, to prevent the patient from inhaling the carbonic acid gas.

Unpublished patent application No. P9102723 describes an apparatus suitable for the treatment of vasoconstriction, the therapeutic effect of which is based on the vasodilator effect of carbonic acid, as well as temperature changes (alternating hot-cold).

Patent application No. P0500508 filed on 19/05/2005, still unpublished, is entitled "Dry carbon dioxide bath in a closed chamber surrounding the lower part of the human body, in which exercises can be done on a leg machine."

The methods referred to above can be well used for the treatment of circulation problems/vasoconstriction, but their disadvantage is that the patient has to go to the appropriate medical institution - by appointment - to have the treatment. In serious cases, especially for patients unable to walk, this may cause great difficulties.

The aim of the invention is to provide a low-cost, easy to use medical appliance for the treatment of mainly vasoconstriction, the use of which is independent of location and provides a safe natural therapy.

Thus the solution according to the invention is a medical appliance for the treatment of mainly vasoconstriction, which is essentially a flexible hose, loosely following the shape of the body, that can be fixed to the body in an airtight manner.
The hose is preferably a stocking fixed near the top of the thigh or under the knee. The material of the stocking is a synthetic plastic not harmful to the organism, not permeable to carbon dioxide gas, from which no substance harmful to the organism can be dissolved by carbon dioxide.

From a pressurized cylinder filled with carbon dioxide gas, through a flexible tube, carbon dioxide gas can be led into the hose. Upon filling with gas the hose can be sealed in an airtight manner and fixed to the body by means of a special self-adhesive tape. The gas diffusing through the skin reaches the tissues and exerts a specific vasodilator effect. This effect is exerted on the skin, the connective tissues under the skin, and on the internal organs as well.

A preferred embodiment of the medical appliance according to the invention is shown in detail in the following figures, where
Figure 1: is a side view of the stocking on a leg, fixed at the top of the thigh and filled with carbon dioxide;
Figure 2: is a side view of the stocking on a leg, fixed under the knee and filled with carbon dioxide.

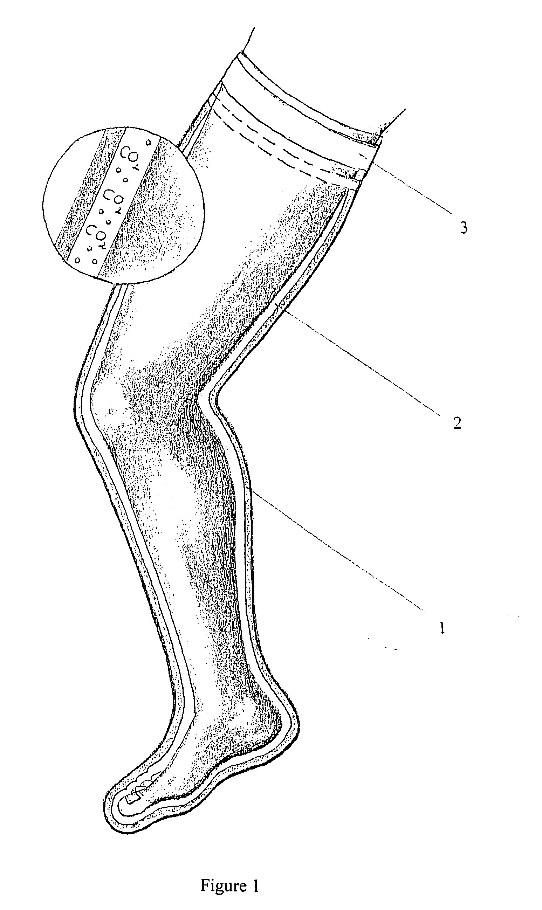

Figure 1 shows the stocking 1 made of a flexible material on a leg, loosely following the shape of the body. The stocking 1 is fixed in an airtight manner at the top of the thigh by means of a special self-adhesive tape 3. The space between the stocking 1 and the surface of the body is filled with carbon dioxide gas 2.

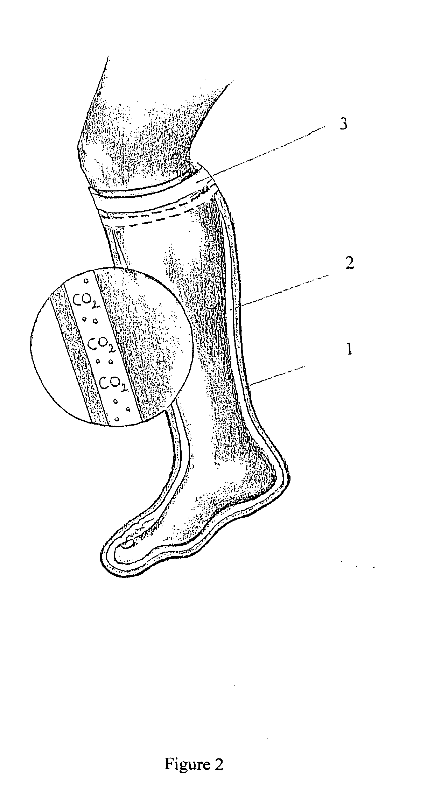

Figure 2 shows the stocking 1 fixed under the knee by means of a special self-adhesive tape 3.

During the treatment from a pressurized plastic cylinder filled with carbon dioxide, through a flexible tube, CO₂ gas can be led into the stocking 1 put on the leg.
Upon filling with the gas the stocking 1 is fixed to the appropriate part of the leg by means of a self-adhesive tape 3. During the treatment the carbon dioxide gas is freely in contact with the surface of the given part of the body, and the gas diffusing through the skin reaches the tissues and exerts its specific vasodilator effect.

The advantages of the medical appliance according to the invention are that it is low-cost, easy to use, its use is independent of location, and provides a safe natural therapy without side effects, even to patients confined to bed.

## Claims

1. A medical appliance for the treatment of mainly vasoconstriction, wherein it is a hose made of a flexible material not permeable to gas, loosely following the shape of the body, which can be filled with carbon dioxide gas (2) and fixed to the body in an airtight manner.

2. A medical appliance according to claim 1, wherein fixing to the body in an airtight manner is solved by means of a special self-adhesive tape (3).

3. A medical appliance according to claims 1 and 2, wherein the hose is a stocking (1) fixed near the top of the thigh.

4. A medical appliance according to claims 1 and 2, wherein the hose is a stocking (1) fixed under the knee.

5. A medical appliance according to claim 1, wherein the material of the hose is a synthetic plastic not harmful to the organism.
